# EUROPEAN PATENT APPLICATION

(11) **EP 2 374 811 A1**
(43) Date of publication of application: **12.10.2011**
(21) Application number: 10013160.6
(22) Date of filing: 23.07.2003
(51) Int. Cl.: C07K 1/00, C07K 16/00, C12P 21/00, C07H 21/04, C12N 15/62

(54) **IgG Fc/HIV-gp120/c3d fusion protein**

(30) Priority: 23.07.2002 US 397605 P
(62) Divisional of application: 03765923.2
(71) Applicant: Duke University, Durham, NC 27708-0083 (US)
(72) Inventor: Haynes, Barton F., Durham NC 27708-0083 (US); Montefiori, David C., Durham NC 27708-0083 (US)
(74) Representative: Atkinson, Peter Birch

(57) **Abstract**

The present invention relates, in general, to an immunogen and, in particular, to an immunogen for inducing antibodies that neutralize a wide spectrum of HIV primary isolates. The invention also relates to a method of inducing anti-HIV antibodies using same.

## Description

The present invention claims priority from Provisional Application No. 60/397,605, filed July 23, 2002, the entire content of which is incorporated herein by reference.

### TECHNICAL FIELD

The present invention relates, in general, to an immunogen and, in particular, to an immunogen for inducing antibodies that neutralize a wide spectrum of HIV primary isolates. The invention also relates to a method of inducing anti-HIV antibodies using such an immunogen.

### BACKGROUND

As the HIV epidemic continues to spread worldwide, the need for an effective HIV vaccine remains urgent. A key obstacle to HIV vaccine development is the extraordinary variability of HIV and the rapidity and extent of HIV mutation (Wain-Hobson in The Evolutionary biology of Retroviruses, SSB Morse Ed. Raven Press, NY, pgs 185-209 (1994)).

Myers, Korber and colleagues have analyzed HIV sequences worldwide and divided HIV isolates into groups or clades, and provided a basis for evaluating the evolutionary relationship of individual HIV isolates to each other (Myers et al (Eds), Human Retroviruses and AIDS (1995), Published by Theoretical Biology and Biophysics Group, T-10, Mail Stop K710, Los Alamos National Laboratory, Los Alamos, NM 87545). The degree of variation in HIV protein regions that contain CTL and T helper epitopes has also recently been analyzed by Korber et al, and sequence variation documented in many CTL and T helper epitopes among HIV isolates (Korber et al (Eds), HIV Molecular Immunology Database (1995), Published by Theoretical Biology and Biophysics Group, Los Alamos National Laboratory, Los Alamos, NM 87545).

A new level of HIV variation complexity was recently reported by Hahn et al by demonstrating the frequent recombination of HIV among clades (Robinson et al, J. Mol. Evol. 40:245-259 (1995)). These authors suggest that as many as 10% of HIV isolates are mosaics of recombination, suggesting that vaccines based on only one HIV clade will not protect immunized subjects from mosaic HIV isolates (Robinson et al, J. Mol. Evol. 40:245-259 (1995)).

The present invention relates to an immunogen suitable for use in an HIV vaccine. The immunogen induces broadly cross-reactive neutralizing antibodies in humans and neutralizes a wide spectrum of HIV primary isolates.

### SUMMARY OF THE INVENTION

The present invention relates to an immunogen for inducing antibodies that neutralize a wide spectrum of HIV primary isolates. The invention also relates to a method of inducing anti-HIV antibodies using such an immunogen.

Objects and advantages of the present invention will be clear from the description that follows.

### BRIEF DESCRIPTION OF THE DRAWINGS

Figure 1. Schematic representation of C3d-89.6 gp120-hIg construct.
Figure 2. Schematic representation of construct of C3d-89.6 gp120-Ig.

### DETAILED DESCRIPTION OF THE INVENTION

The present invention relates to an immunogen that induces broadly reactive neutralizing antibodies that are necessary for an effective AIDS vaccine. The immunogen of the invention comprises at least one fusion protein that comprises at least 3 components: i) an IgG Fc component, ii) an HIV envelope component, and iii) a C3d component. Advantageously, the IgG Fc component is present in the fusion protein N-terminal to the HIV envelope component which is N-terminal to the C3d component. However, the components can be present in virtually any order. In addition, intervening sequences (e.g., linkers) can also be present. The invention further relates to a nucleic acid sequence encoding such a fusion protein.

The IgG Fc (advantageously, human IgG Fc) component of the fusion protein of the invention provides the fusion protein with a longer serum/plasma/extracellular fluid half-life. The C3d (advantageously, human C3d) component targets the fusion protein to antigen presenting cells of the immune system that express CD21 (the C3d receptor) and thereby promotes antigen presentation (see Ahearn et al, Adv. Immunol. 46:183 (1989), Cooper et al, Ann. Rev. Immunol. 6:85 (1988), Dempsey et al, Science 271:348 (1996), Ross et al, AIDS Res. Hum. Retroviruses 17:829 (2001)). The HIV envelope component is, advantageously, HIV-1 gp120, however, HIV-1 gp140, gp160, gp41 and smaller portions of gp120 and gp41 can also be used. The HIV component can include a T-helper epitope and can comprise residues from the C4 domain of HIV gp120 (e.g., about 16 contiguous residues from the C4 domain (for example, about residues 421 to 436) (see also C4 domain sequences in WO 03/039470)). The HIV component can include residues from the V3 domain of gp120 that are selected, for example, so as to include a B cell neutralizing antibody epitope. Advantageously, at least 23 contiguous residues of the V3 domain are used, for example, at least residues 297 to 322 of the V3 domain of HIV gp120. The V3 residues can be selected so as to be representative of higher order structural motifs present in a population, which motifs mediate V3 functions in the course of envelope mediated HIV interaction with host cells. The Los Alamos

National Laboratories Human Retroviruses and AIDS Data base (see, for example, Human Retroviruses and AIDS, 2000, Published by the Theoretical Biology and Biophysics GT-10, Mail StopK710, LANL, Los Alamos, NM) presently contains over 14,000 HIV V3 envelope sequences. Examples of V3 sequences suitable for use in the invention include, but are not limited to, those described in WO 03/039470 and those listed below:

| **V3 Sequence(s)** |
|---|
| RPNNNTRRNIHIGLGRRFYAT |
| RPNNNTRRSVRIGPGGAMFRTG |
| RPIKIERKRIPLGLGKAFYTTK |
| RPSVNNTRRSIHMGLGRAFYTTG |
| RPNRHTGKSIRMGLGLRAWHTTR |
| RRNIHIGLGRRF RRSVRIGPGGAM |
| |
| RKSIRIGPGRAV RRRISIGPGRAF |
| RKSIHIGPGRAF RKSIHIAPGRAF |
| RPNNNTRKGIHIGPGRTFFATG |
| RPNNNTRKSINIGPGRAFYTTG |
| RPNNNTRKSIQIGPGRAFYTTG |
| RPNNNTRKSIHIGPGRAFYTTG |
| RPNNNTRKSIHIGPGRAFYATE |
| RPNNNTRKRMTLGPGKVFYTTG |
| RPGNNTRGSIHLHPGRKFYYSR |

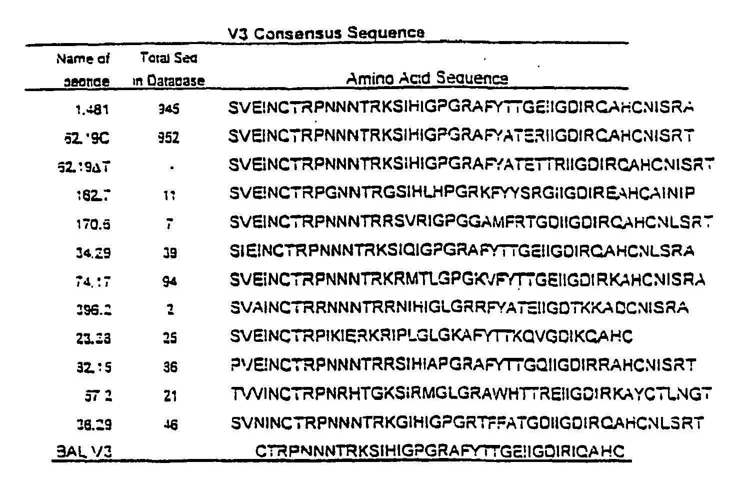

The fusion protein can be used itself as an immunogen or the fusion protein can be present as a complex in which the HIV envelope component has been "activated" to expose intermediate conformations of conserved neutralization epitopes that normally are only transiently or less well exposed on the surface of the HIV virion. The "frozen" triggered form of HIV envelope makes available specific epitopes for presentation to B lymphocytes. The result of this epitope presentation is the production of antibodies that broadly neutralize HIV.

The concept of a triggered envelope is consistent with observations that the gp41 HR-2 region peptide, DP178, can capture an uncoiled conformation of gp41 (Furata et al, Nature Struct. Biol. 5:276 (1998)), and with the report that formalin-fixed HIV-infected cells can generate broadly neutralizing antibodies (LaCasse et al, Science 283:357 (1997)). Recently a monoclonal antibody against the coiled-coil region bound to a conformational determinant of gp41 in HR1 and HR2 regions of the coiled-coil gp41 structure, but did not neutralize HIV (Jiang et al, J. Virol. 10213 (1998)). However, this latter study proved that the coiled-coil region is available for antibody to bind if the correct antibody is generated.

Conserved neutralization sites on the HIV envelope can be on two regions: they can be on gp41 and they can be on gp120.

The regions and conformations of gp41 that are exposed during gp140 (or gp160) "triggering" ("activation") can be expected to be conserved since: i) the amino acid sequences of the coiled-coil region are conserved and ii) the function of the fusogenic envelope complex is conserved and essential for virus pathogenicity. This conservation is key to the production of broadly neutralizing anti-HIV antibodies.

In one embodiment, the fusion protein of the invention has the characteristics of a receptor (CD4)-ligated envelope with CCR5 binding region exposed but unlike CD4-ligated proteins that have the CD4 binding site blocked, this fusion protein has the CD4 binding site exposed (open). Moreover, in this embodiment, the fusion protein can be devoid of host CD4, which avoids the production of potentially harmful anti-CD4 antibodies upon administration to a host.

The gp120 envelope component of the fusion protein of the invention can be ligated with a ligand that binds to a site on gp120 recognized by an A32 monoclonal antibodies (mab) (Wyatt et al, J. Virol. 69:5723 (1995), Boots et al, AIDS Res. Hum. Retro. 13:1549 (1997), Moore et al, J. Virol. 68:8350 (1994)). One A32 mab has been shown to mimic CD4 and when bound to gp120, upregulates (exposes) the CCR5 binding site (Wyatt et al, J. Virol. 69:5723 (1995)). Ligation of gp120 with such a ligand also upregulates the CD4 binding site and does not block CD4 binding to gp120. Advantageously, such ligands also upregulate the HR-2 binding site of gp41 that can be bound to cleaved gp120, uncleaved gp140 and cleaved gp41, thereby further exposing HR-2 binding sites on these proteins - each of which are potential targets for anti-HIV neutralizing antibodies when expressed as IgFc/gp120/C3d fusion proteins.

In a specific aspect of this embodiment, the HIV-1 gp120, gp140 or gp160 component of the fusion protein of the invention is ligated with either an intact A32 mab, a Fab2 fragment of an A32 mab, or a Fab fragment of an A32 mab, with the result that the CD4 binding site, the CCR5 binding site and the HR-2 binding site on gp120, gp140 or gp160 are exposed/upregulated. The immunogen can comprise an A32 mab (or fragment thereof) bound to the gp120 component of the fusion protein or can comprise an A32 mab (or fragment thereof) bound and cross-linked with a cross-linker such as .3% formaldehyde or a heterobifunctional cross-linker such as DTSSP (Pierce Chemical Company) to the gp120 component of the fusion protein. An A32 mab (or fragment thereof) bound to the gp140 or gp120 component of the fusion protein results in upregulation (exposure) of HR-2 binding sites in the gp120 and gp140. Binding of an A32 mab (or fragment thereof) to the gp120 or gp140 components also results in upregulation of the CD4 binding site and the CCR5 binding site. As with complexes comprising gp120 containing fusion proteins, complexes comprising gp140-containing fusion proteins and an A32 mab (or fragment thereof) can be used as an immunogen uncross-linked or cross-linked with cross-linker such as .3% formaldehyde or DTSSP. In one embodiment, the invention relates to an immunogen comprising a fusion protein the gp140 component of which is bound and cross linked to a Fab fragment of an A32 mab, optionally bound and cross-linked to an HR-2 binding protein.

The fusion protein of the invention triggered with a ligand that binds to the A32 mab binding site on gp120 can be administered in combination with at least a second fusion protein comprising a second HIV-1 gp120 (or gp140) component, triggered by a ligand that binds to a site distinct from the A32 mab binding site, such as the CCR5 binding site recognized by mab 17b. The 17b mab (Kwong et al, Nature 393:648 (1998) available from the AIDS Reference Repository, NIAID, NIH) augments sCD4 binding to gp120. This second fusion protein can, for example, comprise an HIV-1 gp120 component ligated with either the whole 17b mab, a Fab2 fragment of the 17b mab, or a Fab fragment of the 17b mab. It will be appreciated that other CCR5 ligands, including other antibodies (or fragments thereof), that result in the CD4 binding site being exposed can be used in lieu of the 17b mab. This further fusion protein can comprise a gp120 component with the 17b mab, or fragment thereof, (or other CCR5 ligand as indicated above) bound thereto or can comprise a gp120 component with the 17b mab, or fragment thereof, (or other CCR5 ligand as indicated above) bound and cross-linked with an agent such as .3% formaldehyde or a heterobifunctional cross-linker, such as DTSSP (Pierce Chemical Company). (This second fusion protein comprising an HIV-1 gp120 component ligated with either the whole 17b mab, a Fab2 fragment of the 17b mab, or a Fab fragment of the 17b mab (or other CCR5 ligand) can itself also be used as an immunogen.)

Soluble HR-2 peptides, such as T649Q26L and DP178, can be added to the above-described complexes to stabilize epitopes on the gp120 or gp140 components of the fusion proteins, and can be administered either cross-linked or uncross-linked with the immunogen complex.

A series of monoclonal antibodies (mabs) have been made that neutralize many HIV primary isolates, including, in addition to the 17b mab described above, mab IgG1b12 that binds to the CD4 binding site on gp120(Roben et al, J. Virol. 68:482 (1994), Mo et al, J. Virol. 71:6869 (1997)), mab 2G12 that binds to a conformational determinant on gp120 (Trkola et al, J. Virol. 70:1100 (1996)), and mab 2F5 that binds to a membrane proximal region of gp41 (Muster et al, J. Virol. 68:4031 (1994)). A mixture fusion proteins comprising triggered envelope components can be used to optimize induction of antibodies that neutralize a broad spectrum of HIV primary isolates. Such fusion proteins, when administered to a primate, for example, either systemically or at a mucosal site, induce broadly reactive neutralizing antibodies to primary HIV isolates.

As indicated above, various approaches can be used to "freeze" fusogenic epitopes in accordance with the invention. For example, "freezing" can be effected by addition of soluble HR-2 peptides, such as the DP-178 or T-649Q26L peptides, that represent portions of the coiled coil region, and that when added to CD4-triggered envelop, result in prevention of fusion (Rimsky et al, J. Virol. 72:986-993 (1998)). HR-2 peptide can be bound (or crosslinked by DTSSP (Pierce Co.), formaldehyde) to the gp140 component of the fusion protein of the invention and the complex can be used as an immunogen. "Freezing" can also be effected by the addition of 0.1% to 3% formaldehyde or paraformaldehyde, both protein cross-linking agents, to the complex, to stabilize the complex (LaCasse et al, Science 283:357-362 (1999)). Further, "freezing" of gp120 fusion intermediates can be effected by addition of heterobifunctional agents such as DSP (dithiobis[succimidylproprionate]) (Pierce Co. Rockford, ILL., No. 22585ZZ) or the water soluble DTSSP (Pierce Co.) that use two NHS esters that are reactive with amino groups to cross link and stabilize the CD4, CCR5 or CXCR4, gp160 complex.

Inherent differences exist in HIV isolates among HIV clades and among HIV isolates from patients in varying geographic locations. Triggered fusion proteins for HIV vaccine development can be made with HIV envelope components from a variety of HIV clades and from a variety of locations. Triggered fusion proteins comprising antibodies or fragments thereof that upregulate the CCR5 binding site, the CD4 binding site, or both, or antibodies, or fragments thereof, that are CD4 inducible can be produced by co-expressing in a dicistronic manner in a plasmid both fusion protein and, for example, the heavy and light chain of the Fab region of the antibody, in order to produce a recombinant protein that has the properties of the above described complexes.

The fusion protein(s) of the invention can be formulated with a pharmaceutically acceptable carrier and/or adjuvant (such as alum) using techniques well known in the art. Suitable routes of administration of the present immunogen include systemic (e.g. intramuscular or subcutaneous). Alternative routes can be used when an immune response is sought in a mucosal immune system (e.g., intranasal). Alternatively, a construct encoding the fusion protein be administered under conditions such that the encoding sequence is expressed and the fusion proein is produced.

Certain aspects of the invention are described in greater detail in the non-limiting Example that follows. (See also WO 02/024149 or Application No. 09/960,717, filed September 24, 2001, and Application No. 10/431,596, filed May 8, 2003 and Provisional Application No. 60/471,327, filed May 19, 2003, which are incorporated herein by reference.)

### EXAMPLE 1

Guinea pigs were immunized with an IgG-89.6gp120-C3d fusion protein. The protein was administered in Complete Freund's Adjuvant (CFA) and then Incomplete Freund's Adjuvant (IFA). The resulting neutralizing antibody (NAb) titers are shown in Table 1. (SHIV-89.6P is the virus against which the neutralizing antibodies were directed.)

| Immunization of Guinea Pigs with IgG-89.6gp120-C3d Protein | | | | |
|---|---|---|---|---|
| Animals were immunized with 50 µg protein in CFA and then IFA. | | | | |
| | | | Nab titer with:* | |
| Animal | Bleed date | Number of inoculations | SHIV-89.6 | SHIV-89.6P |
| GP356 | 03-26-01 | Pre | <10 | <20 |
| | 04-27-01 | 2x | 44 | <20 |
| | 05-18-01 | 3x | 157 | 95/114 |
| | 06-07-01 | 4x | 781 | 110/90 |
| | | | | |
| GP357 | 03-26-01 | Pre | <10 | <20 |
| | 04-27-01 | 2x | 19 | 23 |
| | 05-18-01 | 3x | 49 | <20 |
| | 06-07-01 | 4x | 41 | <20 |
| | | | | |
| GP358 | 03-26-01 | Pre | <10 | <20 |
| | 04-27-01 | 2x | 35 | 30 |
| | 05-18-01 | 3x | 73 | 20 |
| | 06-07-01 | 4x | 61 | 62 |

| | | | | |
|---|---|---|---|---|
| *Nab titers are the reciprocal serum dilution at which 50% of cells were protected from virus-induced killing as measured by neutral red uptake. | | | | |

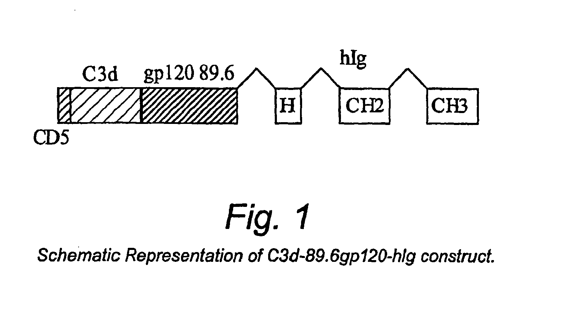

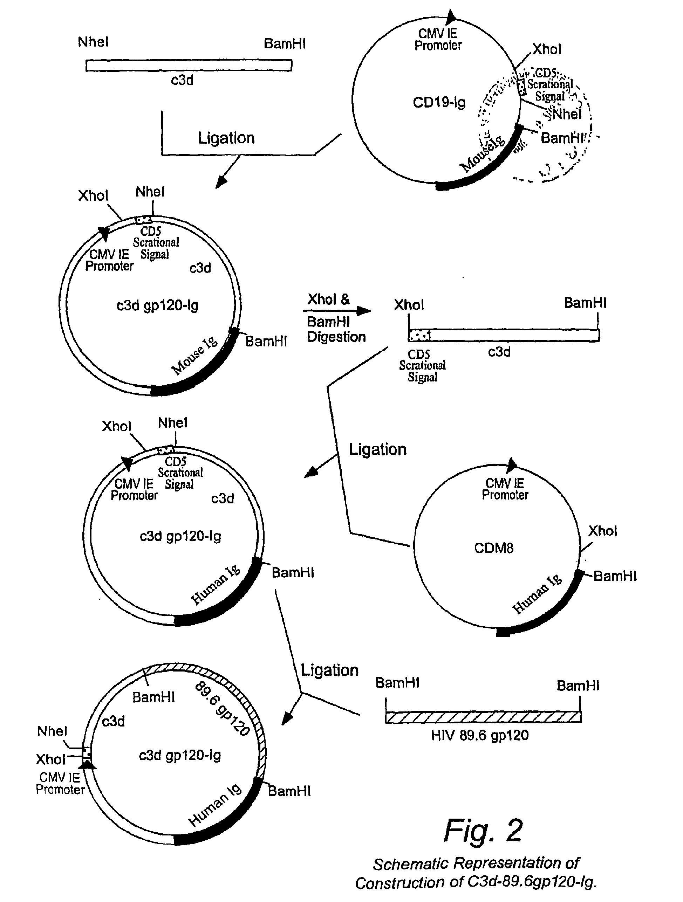

To generate c3d-gp120 89.6 Ig construct (Figure 1), the following steps have been taken utilizing the standard molecular techniques (Figure 2):
1) To facilitate cloning, a 5' primer (Nhel-c3d-3064/F1, CCG TGC TAG CCC ACC TCA TTG TGA CCC CCT CG) with NheI restrict enzyme, and 3' primer (BamH1-c3d-3946/R1: CTA TTG GAT CCC GGC GGC TGG GCA GTT GGA GGG ACA C) with BamHI restriction enzyme site, were derived from complement C3 and synthesized.
2) A DNA fragment containing c3d coding sequence was generated by PCR using plasmid pC3d (from David Montifiori)that contains the entire C3 cDNA as a template and primers Nhe1-c3d-3064/F1, and BamH1-c3d-3946/R1.
3) The PCR product was digested with NheI and BamHI and ligated with plasmid CD-19-Rg containing a CD5 secrational sequence.
4) c3d DNA fragment along with CD5 secrational signal sequence was then cut out from the resulting plasmid with XhoI and BamI, and cloned into XhoI-BamHI sites in CDM8 plasmid that containing human Ig gene.
5) To clone 89.6 gp120, a 5' primer (Bam89.6/F1: CTA TTG GAT CCA GCA AAA GAA AAA ACG TGG GTC ACA ATC) and a 3' primer (Bam89.6gp120R1: CTC TGG ATC CCG TCT TTT TTC TCT TTG CAC TGT TCT TCT C) with BamHI restriction enzyme sites were derived from HIV 89.6 env and synthesized.
6) HIV 89.6 envelope gp120 DNA (From Bob Dome) was generated by PCR using 89.6gp120 plasmid as a template and primers Bam89.6/F1, and Bam89.6gp120R1.
7) HIV 89.6 envelope gp120 PCR product was then digested with BamHI and ligated with BamHI digested-plasmid CDM8-c3d DNA. Clones with the correct orientation of HIV 89.6 envelope gp120 gene was identified by sequencing.

All documents cited above are hereby incorporated in their entirety by reference.

One skilled in the art will appreciate from a reading of this disclosure that various changes in form and detail can be made without departing from the true scope of the invention.

Certain embodiments of the invention are described below.
1. A fusion protein comprising:
   i) an IgG Fc component,
   ii) an HIV envelope component, and
   iii) a C3d component.
2. The protein according to embodiment 1 wherein said IgG Fc component is present in said fusion protein N-terminal to said HIV envelope component.
3. The protein according to embodiment 1 wherein said HIV envelope component is present in said fusion protein N-terminal to said C3d component.
4. The protein according to embodiment 1 wherein said IgG Fc component is present in said fusion protein N-terminal to said HIV envelope component and said HIV envelope component is present in said fusion protein N-terminal to said C3d component.
5. The protein according to embodiment 1 wherein said protein further comprises at least one intervening sequence between at least 2 of said components.
6. The protein according to embodiment 1 wherein said IgG Fc component is a human IgG Fc component.
7. The protein according to embodiment 1 wherein said C3d component is a human C3d.
8. The protein according to embodiment 1 wherein said C3d component targets said fusion protein to antigen presenting cells that express CD21 and thereby promotes antigen presentation.
9. The protein according to embodiment 1 wherein said HIV envelope component is HIV-1 gp120, gp140, gp160, gp41, or immunogenic portion of gp120 or gp41.
10. The protein according to embodiment 1 wherein said HIV envelope component comprises residues of the V3 domain of gp120 and includes a B cell neutralizing antibody epitope.
11. An immunogenic composition comprising at least one of said fusion proteins according to embodiment 1.
12. The composition according to embodiment 11 wherein said composition further comprises a carrier.
13. A complex comprising said fusion protein according to embodiment 1, wherein said HIV envelope component of said fusion protein is activated so that intermediate conformations of conserved neutralization epitopes of said HIV envelope component are exposed.
14. A complex comprising said fusion protein according to embodiment 1 wherein said HIV envelope component is bound to a ligand that upregulates at least one of a CD4 binding site and a CCR5 binding site of said HIV envelope component.
15. The complex according to embodiment 14 wherein said ligand is an antibody, or Fab₂ or Fab fragment thereof.
16. The complex according to embodiment 14 wherein said ligand binds to a CCR5 binding site of said HIV envelope component and upregulates a CD4 binding site of said HIV envelope component.
17. The complex according to embodiment 16 wherein said ligand upregulates a CCR5 and a CD4 binding site on said HIV envelope component.
18. A method of inducing an immune response in a mammal comprising administering to said mammal an amount of said fusion protein according to embodiment 1 sufficient to effect said induction.
19. A nucleic acid sequence encoding said fusion protein according to embodiment 1.
20. An expression vector comprising the nucleic acid according to embodiment 19 operably linked to a promoter.

## Claims

1. A fusion protein comprising:
i) an IgG Fc component,
ii) an HIV envelope component, and
iii) a C3d component.

2. The protein according to claim 1 wherein said IgG Fc component is present in said fusion protein N-terminal to said HIV envelope component.

3. The protein according to claim 1 wherein said HIV envelope component is present in said fusion protein N-terminal to said C3d component.

4. The protein according to claim 1 wherein said IgG Fc component is present in said fusion protein N-terminal to said HIV envelope component and said HIV envelope component is present in said fusion protein N-terminal to said C3d component.

5. The protein according to claim 1 wherein said protein further comprises at least one intervening sequence between at least 2 of said components.

6. The protein according to claim 1 wherein said IgG Fc component is a human IgG Fc component.

7. The protein according to claim 1 wherein said C3d component is a human C3d.

8. The protein according to claim 1 wherein said C3d component targets said fusion protein to antigen presenting cells that express CD21 and thereby promotes antigen presentation.

9. The protein according to claim 1 wherein said HIV envelope component is HIV-1 gp120, gp140, gp160, gp41, or immunogenic portion of gp120 or gp41.

10. The protein according to claim 1 wherein said HIV envelope component comprises residues of the V3 domain of gp120 and includes a B cell neutralizing antibody epitope.

11. An immunogenic composition comprising at least one of said fusion proteins according to claim 1, said composition preferably further comprising a carrier.

12. A complex comprising said fusion protein according to claim 1, wherein said HIV envelope component of said fusion protein is activated so that intermediate conformations of conserved neutralization epitopes of said HIV envelope component are exposed.

13. A complex comprising said fusion protein according to claim 1 wherein said HIV envelope component is bound to a ligand that upregulates at least one of a CD4 binding site and a CCR5 binding site of said HIV envelope component, said ligand preferably being one that binds to a CCR5 binding site of said HIV envelope and upregulates a CD4 binding site of said HIV envelope component and preferably upregulates a CCR5 and a CD4 binding site on said HIV envelope component, said ligand preferably being an antibody, or Fab₂ or Fab fragment thereof.

14. A fusion protein according to claim 1 for use in inducing an immune response in a mammal.

15. A nucleic acid sequence encoding said fusion protein according to claim 1, said nucleic acid preferably being incorporated in an expression vector and operably linked to a promoter.
